# EUROPÄISCHE PATENTSCHRIFT

(11) **EP 2 091 640 B1**
(45) Veröffentlichungstag und Bekanntmachung des Hinweises auf die Patenterteilung: **30.06.2010**
(21) Anmeldenummer: 07815193.3
(22) Anmeldetag: 22.11.2007
(51) Int. Cl.: B01F 3/14, B01F 7/16, B01F 15/02, B29B 7/94, B29C 47/10

(54) **VERFAHREN UND VORRICHTUNG ZUR EINBRINGUNG VON ZUSATZSTOFFEN**
METHOD AND DEVICE FOR INTRODUCING ADDITIVE MATERIALS
PROCÉDÉ ET DISPOSITIF D'INTRODUCTION D'ADDITIFS

(30) Priorität: 23.11.2006 AT 19512006
(43) Veröffentlichungstag der Anmeldung: 26.08.2009
(60) Teilanmeldung: 10003411.5
(73) Patentinhaber: EREMA Engineering Recycling Maschinen und Anlagen Gesellschaft m.b.H., 4052 Ansfelden (AT)
(72) Erfinder: WENDELIN, Gerhard, 4030 Linz (AT); HACKL, Manfred, 4040 Linz-Urfahr (AT); FEICHTINGER, Klaus, 4040 Linz (AT)
(74) Vertreter: Wildhack, Andreas
(86) Internationale Anmeldenummer: PCT/AT2007/000527
(87) Internationale Veröffentlichungsnummer: WO 2008/061269

(56) Entgegenhaltungen:
- DE-A1- 2 134 305
- DE-U1- 20 011 402
- GB-A- 728 323
- GB-A- 778 953

## Beschreibung

Die Erfindung betrifft ein Verfahren gemäß dem Oberbegriff des Anspruchs 1 sowie eine Vorrichtung zur Durchführung des Verfahrens gemäß dem Oberbegriff des Anspruchs 9.

Aus dem Stand der Technik sind zahlreiche Verfahren und Vorrichtungen bekannt, bei denen die flüssigen Zusatzstoffe entweder von oben auf das Kunststoffmaterial aufgesprüht werden oder die Zugabe im Wirbelschichtverfahren erfolgt.

Aus der US 4,522,957 ist es bekannt, flüssige Zusatzstoffe zu Kunststoffgranulat in einem Mischer hinzuzufügen.

In der WO 00/38895 wird ein derartiges Verfahren zur Verminderung der Staubbelastung bzw. Staubentstehung weiter verbessert, indem in einem ersten Schritt der flüssige Zusatzstoff in einer Sprühkammer auf die Plastikgranulate im Gegenstromverfahren aufgedüst wird und anschließend ein statisches Mischverfahren erfolgt.

In der EP 7624 wird ein flüssiger Zusatzstoff zu Kunststoffgranulat zugesetzt, und zwar in einem inerten Gasstrom.

In der WO 84/02530 werden die Kunststoffgranulate zuerst in einem Durchlaufmischer verwirbelt und werden in diesem verwirbelten Zustand im Gasstrom mit hocherhitztem flüssigen Zusatzstoff benetzt.

Aus der WO 9425509 ist ein Verfahren bekannt, bei dem ein Polymergranulat in einer Mischeinrichtung mit einem flüssigen Additiv über eine Einspritzdüse benetzt wird, wobei zur besseren Benetzung die Oberfläche des Kunststoffgranulates unregelmäßig strukturiert bzw. aufgeraut ist.

In der WO 2006/010291 wird ein Verfahren und eine Mischvorrichtung beschrieben, bei denen ein flüssiges Additiv über eine Einspritzvorrichtung in einer Mischeinrichtung einem Kunststoffgranulat zugedüst wird und danach die Mischung in einen Extruder gelangt.

Weiters ist aus der EP 9817 ein Verfahren bekannt, bei dem Kunststoffgranulat zuerst mit einem "coupling agent" bzw. Träger benetzt wird, der eine bessere Verteilung der flüssigen Additive auf der Oberfläche des Kunststoffgranulats sicherstellen soll. Als coupling agents werden insbesondere Paraffine bzw. paraffinartige Substanzen angeführt.

Weiters ist aus der US 4,703,093 ein Verfahren bekannt, bei dem ein flüssiger Zusatzstoff zu einem bereits vorgewärmten Kunststoffgranulat zugesetzt wird.

Die DE 263 16 22 beschreibt ein Verfahren zum gleichzeitigen und kontinuierlichen Zuführen von pulverförmigen Feststoffen und Flüssigkeiten in Behandlungsmaschinen. Dies geschieht über eine Ringdüse, wobei die Flüssigkeit zu einem schlauchartigen Mantel geformt wird, in dessen Zentrum die Feststoffe eingegeben werden.

Derartige Verfahren sind jedoch in erster Linie nur für dünnflüssige, fein zerstäubbare Zusatzstoffe geeignet und funktionieren für höherviskose, zähflüssige Zusatzstoffe oder für Zusatzstoffe von fester oder halbfester Konsistenz nur ungenügend. Meist wird das Kunststoffmaterial nur unvollständig und ungleichmäßig benetzt.

Werden höherviskose Zusatzstoffe auf höhere Temperaturen erhitzt, um dennoch in dünnflüssiger Form zugegeben werden zu können, so bilden sich an kühleren Stellen oder kälteren Oberflächen der Vorrichtung oft Ablagerungen oder Niederschläge der Zusatzstoffe aus.

Dies führt zu Schwierigkeiten und Ungenauigkeiten bei der Dosierung und zur Verschmutzung der Vorrichtungen.

In der GB-A-728 323 werden ein Verfahren gemäß den Oberbegriff des Anspruche 1 und eine Vorrichtung gemäß dem Oberbegriff des Anspruchs 9 offenbart.

Aufgabe der Erfindung ist es, ein Verfahren und eine Vorrichtung zu schaffen, durch das/die nicht-trocken-teilchenförmige, höherviskose Zusatzstoffe zu einem vorgelegten stückigen Material, insbesondere einem Kunststoffmaterial bzw. von Polymerteilchen, einfach und gleichmäßig zugegeben werden können. Die Oberfläche des Materials soll dabei möglichst vollständig und gleichmäßig mit den Zusatzstoffen benetzt werden und die Zusatzstoffe sollen innerhalb der Materialteilchen gleichmäßig verteilt bzw. dispergiert werden. Außerdem sollen die Zusatzstoffe in korrekten Dosierungen zugegeben und Ablagerungen und somit Verschmutzungen an ungewünschten Stellen vermieden werden können.

Diese Aufgaben werden durch die kennzeichnenden Merkmale der Ansprüche 1 bzw. 9 gelöst.

Durch das erfindungsgemäße Verfahren bzw. durch die erfindungsgemäße Vorrichtung wird es ermöglicht, Zusatzstoffe bzw. Beschichtungsstoffe in vorteilhafter Weise auf stückige, teilchenförmig vorliegende Materialien sehr gleichmäßig und homogen aufzubringen, sodass sich eine vollständige Benetzung der Oberfläche der Materialteilchen ergibt.

Außerdem können so auch geringste Mengen an Zusatzstoffen genau dosiert werden, da die gesamte Menge der eingesetzten Zusatzstoffe direkt in die Materialteilchen eingebracht wird und es für die Zusatzstoffe keine Möglichkeit gibt, sich abzulagern. Dies wird insbesondere dadurch gewährleistet, dass die Zusatzstoffe mit keinen kälteren Bauteilen des Behälters bzw. Reaktors in Berührung kommen. Es kommt zu keinerlei Verschmutzungen oder Materialablagerungen von kondensierten bzw. erstarrten Zusatzstoffen an unerwünschte Stellen im Reaktor, wodurch eine oftmalige Reinigung nicht mehr notwendig ist. Der Niederschlag von Zusatzstoffen oder von Staub mit Zusatzstoffen an kühleren Stellen wird im Vergleich zu der aus dem Stand der Technik bekannten Eindosierung durch Aufsprühen der Zusatzstoffe auf die Materialteichen von oben stark verringert oder sogar verhindert.

Die dynamische Bewegung bzw. Rotation der Materialteilchen im Behälter erleichtert die Einbringung der Zusatzstoffe, das Aufbringen auf die Oberflächen der Materialteilchen und unterstützt die gleichmäßige Verteilung bzw. Dispergierung der Zusatzstoffe auf die Materialteilchen. Dies wird dadurch gewährleistet, dass die Materialteilchen an der Innenseite der Seitenwand des Behälters entlanggleiten bzw. vorbeirotieren und auf diese Weise die dort austretenden Zusatzstoffe mitnehmen bzw. mitreißen.

Durch das erfindungsgemäße Verfahren wird somit die gesamte Oberfläche benetzt und die Zusatzstoffe in der Mischung der Teilchen optimal verteilt.

Insbesondere für sehr hochviskose Zusatzstoffe ist es vorteilhaft, wenn die Zugabeeinrichtungen in demjenigen Bereich bzw. in derjenigen Höhe der Innenseite des Behälters angeordnet sind, auf den bzw. der die bewegten bzw. rotierenden Materialteilchen den höchsten Druck ausüben. Dadurch wird eine gute Verteilung des Materials sichergestellt. Dieser Bereich bzw. der vom Material auf die Seitenwand ausgeübte Druck wird in Abhängigkeit von der Drehzahl, der Art, Anzahl und Form der Mischwerkzeuge bestimmt.

Weitere Vorteile der Erfindung finden sich in den abhängigen Ansprüchen.

Es können eine oder mehrere Zugabeeinrichtungen vorgesehen sein. Diese Zugabeeinrichtungen sind an der Innenseite der Seitenwand des Behälters angeordnet bzw. münden an der Innenfläche der Seitenwand des Behälters in den Behälter ein.

Für die Anbringung und Anordnung der Zugabeeinrichtungen bestehen unterschiedliche Möglichkeiten. Besonders vorteilhaft ist die Vorsehung mehrerer Zugabeeinrichtungen, die beispielsweise auf gleicher Höhe über dem Behälterboden bzw. dem Mischwerkzeug angeordnet sind und über den Umfang der Innenwand des Behälters vorzugsweise gleichmäßig verteilt sind.

Eine weitere Möglichkeit besteht darin, die einzelnen Zugabeeinrichtungen in einer geraden vertikalen Reihe oder einer schräg nach oben verlaufenden Reihe übereinander, gegebenenfalls versetzt oder spiralförmig, anzuordnen. Auch können die Zugabeeinrichtungen, insbesondere statistisch oder gleichmäßig, im Behälter verteilt sein oder es kann nur eine einzige Zugabeeinrichtungen vorgesehen sein.

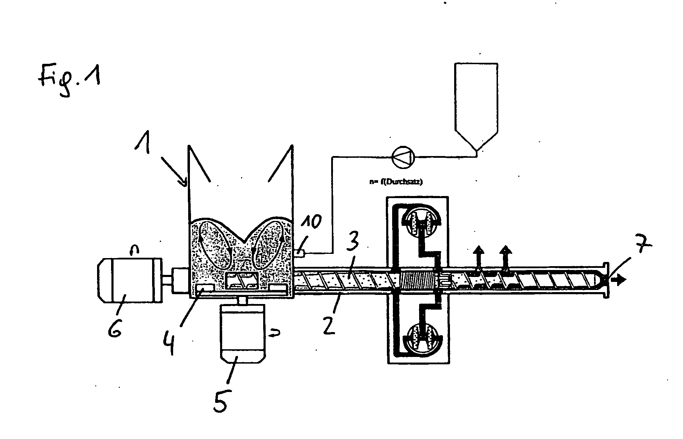

Die Zugabeeinrichtungen sind im Behälter so angeordnet, dass sie, insbesondere ständig und permanent, unterhalb des Niveaus des im Behälter befindlichen Materials liegen, sodass die Zugabe der Zusatzstoffe ausschließlich direkt in die Menge der rotierenden Materialteilchen erfolgt. Zumeist wird durch die Bewegung der Materialteilchen im Inneren des Behälters eine Mischthrombe ausgebildet, die schematisch auch in Fig. 1 dargestellt ist. Der Rand bzw. das oberste Niveau der Mischthrombe sollte, vorteilhafterweise während des gesamten Verfahrens, oberhalb der Zugabeeinrichtungen liegen.

Die Zugabeeinrichtungen sind vorteilhafterweise in Höhe des mittleren Drittelbereichs des Füllstandes des Materials im Behälter bzw. der Mischthrombe angeordnet, wodurch eine gleichmäßige Dispergierung der Zusatzstoffe auf die Materialteichen erfolgt.

Die Zugabeeinrichtungen können als einfache Zugabestutzen oder Zugabeöffnungen in der Seitenwand des Behälters ausgebildet sein oder auch in Form von Zugabedüsen ausgestaltet sein. Die Dosierung bzw. Beschickung der Zusatzstoffe erfolgt vorteilhafterweise über Dosierpumpen, beispielsweise Zahnradpumpen oder Membranpumpen. Diese steuern die Menge der zugegebenen Zusatzstoffe. Da, wie beschrieben, die gesamten Zusatzstoffe direkt in die Materialteilchen eingebracht werden können, kann sehr exakt und verlustfrei dosiert werden. Verluste durch Ablagerungen od. dgl. sind weitgehend ausgeschlossen.

Um die Bewegung der Materialteilchen im Inneren des Behälters nicht zu stören, ist es vorteilhaft, wenn die Zugabeeinrichtungen bündig mit der Innenwand des Behälters abschließen und nicht ins Innere des Behälters hineinragen bzw. abstechen.

Meist tritt der Zusatzstoff, der in das Material eingebracht werden soll, in Tröpfchenform oder in pastöser Form aus den Zugabeeinrichtungen aus. Die Materialteilchen bewegen sich durch die durch die Mischwerkzeuge erzwungene Bewegung entlang der Innenwand des Behälters, berühren diese und reiben an dieser. Dadurch werden durch die Materialteilchen die durch die Zugabeeinrichtung gerade austretenden Zusatzstoffe unmittelbar und direkt mitgerissen und verteilen sich umso besser in der Mischung.

Für manche Zusatzstoffe kann eine Benetzung der Behälterwand durch die Zusatzstoffe erwünscht sein, um dadurch eine bessere Dispergierung der Zusatzstoffe mit den Materialteilchen zu bewirken. Um dies zu ermöglichen, kann eine zusätzliche separate Heizvorrichtung vorgesehen werden, die lediglich die Innenseite der Seitenwand des Behälters bzw. die Behälterseitenwand erwärmt. Diese Heizeinrichtung ist vorteilhafterweise unabhängig von temperierbaren bzw. beheizbaren Mischwerkzeugen oder weiteren Heizvorrichtungen zur Erwärmung des Materials im Behälter. Auf diese Weise verringert sich die Viskosität der Zusatzstoffe, wodurch die Fließfähigkeit der Zusatzstoffe verbessert wird und die Innenwand des Behälters besser benetzt ist. Dadurch ist eine noch bessere Verteilung der Zusatzstoffe auf die Materialteilchen gegeben.

Um Verstopfungen von pastösen oder hochviskosen Zusatzstoffen zu vermeiden, können auch die Zugabeeinrichtungen selbst sowie deren Zuleitungen oder Vorratsbehälter beheizbar sein. Auf diese Weise können beispielsweise bei Raumtemperatur feste bzw. pastöse Wachse in ausreichend fluidisierter Form zugegeben werden. Allenfalls kann auch der Druck, der auf die Zusatzstoffe ausgeübt wird bzw. mit dem die Zusatzstoffe zugeführt werden, entsprechend eingestellt, insbesondere in entsprechender Höhe gewählt, werden, um höherviskose Zusatzstoffe einbringen zu können.

Grundsätzlich ist es vorteilhaft, das Material bei erhöhter Temperatur zu behandeln bzw. die Temperatur des Materials eher höher zu halten, da dadurch die Viskositäten der Zusatzstoffe herabgesetzt werden und eine bessere Verteilung und homogenere Dispergierung der Materialteichen erfolgt.

Weiters kann es vorteilhaft sein, eine Benetzung der Innenwand des Behälters durch die Zusatzstoffe zu vermeiden bzw. zu verringern. Dies kann beispielsweise durch besondere Beschichtungen oder auch durch besondere Prägungen der Behälterwand erfolgen. Auf diese Weise können sich die auf der Behälterinnenwand anhaftenden Zusatzstoff-Tröpfchen besser von der Seitenwand lösen und leichter vom Material bzw. von den bewegenden Materialteilchen mitgenommen werden und eine Benetzung der Behälterwand unterbleibt.

Weitere Vorteile und Ausgestaltungen der Erfindung ergeben sich aus der Beschreibung und der beiliegenden Zeichnung.

Die Erfindung ist anhand von Ausführungsbeispielen im der Zeichnung schematisch dargestellt und wird im Folgenden unter Bezugnahme auf die Zeichnung beispielsweise beschrieben.

Fig. 1 zeigt eine schematische Ansicht einer erfindungsgemäßen Vorrichtung.

In Fig. 1 ist eine erfindungsgemäße Vorrichtung in schematischer Schnittansicht dargestellt. Derartige Vorrichtungen sind aus dem Stand der Technik in verschiedenen Ausführungsformen bekannt.

Die in Fig.1 dargestellte Vorrichtung in Form eines Schneidverdichters weist einen Aufnahmebehälter 1 auf, in dessen Bodenbereich ein um eine etwa vertikale Achse verdrehbares Zerkleinerungs- bzw. Mischwerkzeug 4 vorgesehen ist, das von einem Antriebsmotor 5 angetrieben wird. Auf der Höhe dieses Zerkleinerungs- und Mischwerkzeugs 4 ist in der Seitenwand des Aufnahmebehälters 1 eine Öffnung vorgesehen, an die das Gehäuse 2 eines Schneckenextruders angeschlossen ist. Im Gehäuse 2 befindet sich eine Extruderschnecke 3, die von einem Antriebsmotor 6 angetrieben wird. Das durch den Schneckenextruder geförderte, zerkleinerte und gemischte Material, insbesondere ein Kunststoffgut, tritt durch die Austrittsöffnung 7 aus dem Schneckengehäuse aus. Bei der Verarbeitung von Kunststoffmaterial findet ein Aufschmelzen bzw. Plastifizieren des Materials erst im Extruder statt. Der Behälter 1 kann auch mit Vakuum beaufschlagt werden.

Im Aufnahmebehälter 1 wird das zu behandelnde Material vorgelegt. Das Material liegt im Aufnahmebehälter 1 in stückiger bzw. teilchenförmiger Form vor und hat damit im Verhältnis zu seinem Volumen eine große Oberfläche. Das Material kann beispielsweise thermoplastisches Kunststoffmaterial in Form von Flakes, Granulaten, Folienabfällen od. dgl. sein. Auch denkbar sind Holzfasern, Zeitungspapier od. dgl.. Durch die durch das Mischwerkzeug 4 verursachte stete dynamische Bewegung bzw. Rotation der Materialteilchen im Aufnahmebehälter 1 erfolgt eine Durchmischung der einzelnen Teilchen, sowie gegebenenfalls, je nach Ausbildung des Mischwerkzeuges 4, auch eine Zerkleinerung und/oder Vorverdichtung und gegebenenfalls auch eine Erwärmung des Materials bzw. eine Trocknung bzw. Kristallisation. Die Bewegung der Materialteilchen im Aufnahmebehälter 1 dient, insbesondere bei Kunststoffmaterialien, dazu, dass bei Erwärmung die einzelnen Kunststoffteilchen nicht zusammenkleben und dass der stückige Charakter des Materials erhalten bleibt.

Weiters ist im unteren Bereich der Seitenwand des Behälters 1 eine Zugabeeinrichtung 10 in Form eines Zugabestutzens vorgesehen, der über eine Öffnung in den Behälter 1 einmündet, wobei die Öffnung bündig mit der Innenfläche der Seitenwand abschließt und kein Teil der Zugabeeinrichtung 10 ins Innere des Behälters vorsteht. Über diese Zugabeeinrichtung 10 können ein oder mehrere Zusatzstoffe bzw. Beschichtungsstoffe in den Aufnahmebehälter 1 zudosiert werden.

Die Zugabeeinrichtung 10 ist so ausgestaltet, dass sie für die Zugabe von bzw. nicht-trocken-teilchenförmigen bzw. nicht-trocken-pulverförmigen bzw. nicht-trockengranulatförmigen bzw. nicht-trocken-kristallinen Zusatzstoffen geeignet ist. Trockene pulver- oder granulatförmige Zusatzstoffe, wie beispielsweise Pigmente, Füllstoffe od. dgl., werden zumeist über einfache Zugabetrichter von oben zugegeben. Über die Zugabeeinrichtung 10 werden somit pumpfähige dünn- oder zähflüssige, feste, halbfeste oder pastöse Zusatzstoffe, insbesondere von höherer Viskosität, zudosiert. Die Zugabeeinrichtung 10 ist beispielsweise geeignet, dünnflüssige Zusatzstoffe, wie Weichmacher, Peroxide, etc. zähflüssige Zusätze oder auch pastöse, eher festere Zusatzstoffe mit cremeartiger oder teigiger Konsistenz, wie beispielsweise Fette oder Wachse oder auch Polymere zuzugeben. Unter den Begriff der festen Zusatzstoffe sind beispielsweise Wachse oder Fette zu subsumieren, die bei Raumtemperatur zwar formstabil, jedoch immer noch duktil und verformbar sind. Auch ursprünglich pulverförmige Zusätze bzw. Zuschlagstoffe, wie Pigmente, Füllstoffe od. dgl. können mittels einer Trägerlösung als Dispersion oder Aufschlämmung, allenfalls auch als Suspension oder Emulsion, auf diese Weise zugegeben werden.

Die Zugabeeinrichtung 10 ist gemäß Fig. 1 unterhalb des Niveaus der im Behälter 1 befindlichen, rotierenden Materialteilchen bzw. unterhalb des obersten Randes der Mischthrombe angeordnet. Die Zugabe der Zusatzstoffe erfolgt somit nicht von oben, beispielsweise durch Aufsprühen oder Zutropfen, sondern erfolgt durch die Seitenwand des Behälters 1. Dabei wird die Zugabeeinrichtung 10 bzw. die Zugabeöffnung immer von sich vorbeibewegendem Material überstrichen und die austretenden Zusatzstoffe werden mitgerissen und so auf die Materialteilchen aufgebracht und innerhalb der Materialteilchen dispergiert bzw. verteilt.

Die Dispergierung der Zusatzstoffe funktioniert umso besser, je größer die Oberflächen der Materialteilchen sind.

Die Zusatzstoffe, insbesondere reaktive Zusatzstoffe, werden je nach Verdünnungsgrad durch einen allfälligen Träger der Zusatzstoffe, in Mengen zwischen 0,01 und 20 Gew.-% zugegeben. Beispielsweise wird bei der Verwendung von PET-Flakes als Vorlagematerial eine Menge von 0,2 bis 0,6 % eines Zusatzstoffes aufgebracht.

Die maximale Menge, mit der die Zusatzstoffe eingesetzt werden sollten, ist diejenige Menge, die notwendig ist, um die Gesamtoberfläche des im Behälter 1 befindlichen Materials bzw. die Gesamtoberfläche der Materialteilchen zu benetzen.

Je nach der Art des eingesetzten Zusatzstoffes und dessen Reaktivität findet eine Reaktion des Zusatzstoffes mit dem Material unter Umständen erst im Extruder bzw. in der Schmelze statt.

Das Material wird abschließend im Extruder vollständig aufgeschmolzen und wenn nötig filtriert und/oder entgast.

Das erfindungsgemäße Verfahren kann einstufig geführt werden, aber auch in einen zwei- oder mehrstufigen Prozess eingebunden sein. Die Zusatzstoffe werden dabei vorteilhafterweise bereits in der ersten Stufe, in einem vorgeschalteten Vorbehandlungsbehälter bzw. einem ersten Aufnahmebehälter 1 zugegeben. Die Zugabeeinrichtungen sind zu diesem Zweck in diesem Vorbehandlungsbehälter angeordnet. Die weitere Behandlung des Materials und/oder die Zugabe weiterer Zusatzstoffe oder eine allfällige Trocknung bzw. Kristallisation erfolgen dann in weiteren Behältern 1.

## Patentansprüche

1. Verfahren zur Einbringung bzw. Zugabe von nicht-trocken-teilchenförmigen, insbesondere nicht-trocken-pulverförmigen, Zusatzstoffen bzw. Beschichtungsstoffen mit flüssiger, fester, halbfester oder pastöser Konsistenz, gegebenenfalls in suspendierter oder emulgierter Form, insbesondere mit höherer Viskosität, wie beispielsweise Peroxiden, Fetten, Wachsen, IV-Verbesserem, Polymeren od. dgl., zu einem, in einem Aufnahmebehälter bzw. Schneidverdichter (1) bewegten, gemischten und gegebenenfalls erwärmten und zerkleinerte, stückig bzw. teilchenförmig vorliegenden Material, insbesondere Polymerteilchen bzw. -flakes, Holzfasern, Papierschnitzeln od. dgl., wobei die Zusatzstoffe unterhalb des Niveaus des im Behälter (1) befindlichen Materials bzw. der Materialteilchen zugegeben werden, **dadurch gekennzeichnet, dass** die Zusatzstoffe, insbesondere sehr hochviskose Zusatzstoffe, in denjenigen Bereich bzw. in derjenigen Höhe des Behälters (1) zugegeben werden, in dem die, insbesondere im Behälter (1) rotierenden, Materialteilchen den höchsten Druck auf die Seitenwand des Behälters (1) ausüben.

2. Verfahren nach Anspruch 1, **dadurch gekennzeichnet, dass** die Zusatzstoffe in den mittleren Drütelbereich des Füllstandes des Materials im Behälter (1) bzw. einer durch die Rotation ausgebildeten Mischthrombe, zugegeben werden.

3. Verfahren nach einem der Ansprüche 1 bis 2, **dadurch gekennzeichnet, dass** die Zusatzstoffe über eine oder mehrere, an der Innenseite der Seitenwand des Behälters (1) angeordnete bzw. durch die Seitenwand einmündende, insbesondere in gleicher Höhe umfänglich verteilte oder in einer Reihe übereinanderliegende, als Zugabeöffnungen oder Düsen ausgebildete, gegebenenfalls über Dosierpumpen, beispielsweise Zahnradpumpen oder Membranpumpen beschickte, Zugabeeinrichtung(en) (10) zugegeben werden.

4. Verfahren nach einem der Ansprüche 1 bis 3, **dadurch gekennzeichnet, dass** das Niveau der Materialteilchen bzw. der durch die Bewegung ausgebildeten Mischthrombe im Behälter (1) so gehalten wird, dass es ständig oberhalb der Zugabeeinrichtung(en) (10) liegt.

5. Verfahren nach einem der Ansprüche 1 bis 4, **dadurch gekennzeichnet, dass** die Zusatzstoffe durch die bzw. mittels der an der Innenseite der Seitenwand des Behälters (1) und an den Zugabeeinrichtungen (10) entlang- bzw. vorbeistreifenden bzw. - rotierenden Materialteilchen mitgenommen bzw. eingebracht werden bzw. die Zusatzstoffe auf diese Weise auf die Materialteilchen aufgebracht werden.

6. Verfahren nach einem der Ansprüche 1 bis 5, **dadurch gekennzeichnet, dass** vor und/oder während der Zugabe der Zusatzstoffe, unabhängig von der Temperatur des im Behälter (1) befindlichen Materials, die die Innenseite bzw. die Seitenwand des Behälters (1) zusätzlich und separat erwärmt wird, um die Viskosität der zugeführten Zusatzstoffe zu vermindern und die Benetzung zu erhöhen.

7. Verfahren nach einem der Ansprüche 1 bis 6, **dadurch gekennzeichnet, dass** die Zusatzstoffe, insbesondere reaktive Zusatzstoffe, je nach Verdünnungsgrad durch einen allfälligen Träger der Zusatzstoffe, in Mengen zwischen 0,01 und 20 Gew%, bezogen auf das Gesamtgewicht des Endproduktes, zugegeben werden.

8. Verfahren nach einem der Ansprüche 1 bis 7, **dadurch gekennzeichnet, dass** die Zusatzstoffe maximal bis zu einer Menge zugesetzt werden, die nötig ist, um die Gesamtoberfläche des im Behälter (1) befindlichen Materials bzw. der Materialteilchen zu benetzen.

9. Vorrichtung zur Durchführung des Verfahrens nach einem der Ansprüche 1 bis 8, mit zumindest einem Aufnahmebehälter bzw. Schneidverdichter (1), in dem zumindest ein, insbesondere um eine vertikale Achse drehbares, das zu behandelnde stückige bzw. teilchenförmige Material, insbesondere ein Kunststoffmaterial in Form von nicht geschmolzenen Polymerteilchen, Holzfasern, Papierschnitzeln od. dgl., bewegende bzw. im Rotation versetzende, mischende, erwärmende bzw. gegebenenfalls zerkleinernde, Mischwerkzeug (4) angeordnet ist, wobei zumindest eine Zugabeeinrichtung (10) für nicht-trocken-teilchenförmige Zusatzstoffe mit flüssiger, fester, halbfester oder pastöser Konsistenz, gegebenenfalls in suspendierter oder emulgierter Form, insbesondere mit höherer Viskosität, beispielsweise Peroxide, Fette, Wachse, IV-Verbesserer, Polymere od. dgl., vorgesehen ist, wobei die Zugabeeinrichtung (10) unterhalb des Niveaus des im Betrieb im Behälter (1) befindlichen Materials bzw. der Materialteilchen angeordnet ist, **dadurch gekennzeichnet, dass** die Zugabeeinrichtungen (10) in demjenigen Bereich des Behälters (1) angeordnet sind, in dem die, vorzugsweise bewegten, insbesondere im Behälter (1) rotierenden, Materialteilchen den höchsten Druck auf die Seitenwand des Behälters (1) ausüben.

10. Vorrichtung nach Anspruch 9, **dadurch gekennzeichnet, dass** die Zugabeeinrichtungen (10) an der Innenseite der Seitenwand des Behälters (1) angeordnet sind bzw. in der Seitenwand des Behälters (1) in den Behälter (1) einmünden bzw. ausgebildet sind.

11. Vorrichtung nach Anspruch 9 oder 10, **dadurch gekennzeichnet, dass** die Zugabeeinrichtungen (10) auf gleicher Höhe über den Umfang der Innenwand des Behälters (1), vorzugsweise gleichmäßig, verteilt oder in einer Reihe übereinanderliegend, angeordnet sind.

12. Vorrichtung nach einem der Ansprüche 9 bis 11, **dadurch gekennzeichnet, dass** die Zugabeeinrichtungen (10) im Behälter (1) in einer Höhe bzw. in einem Abstand zum Boden bzw. zum Mischwerkzeug (4) angeordnet sind, in der/dem die Zugabeeinrichtungen (10) ständig unterhalb des verfahrensmäßig vorgegebenen Füllstandes der im Behälter (1) befindlichen bzw. rotierenden Materialteilchen bzw. des Niveaus der bei einer Bewegung bzw. Rotation der Materialteilchen ausgebildeten Mischthrombe liegen.

13. Vorrichtung nach einem der Ansprüche 9 bis 12, **dadurch gekennzeichnet, dass** die Zugabeeinrichtungen (10) in Höhe des mittleren Drittelbereichs des verfahrensmäßig vorgegebenen Füllstandes des Materials im Behälter (1) bzw. der Mischthrombe angeordnet sind.

14. Vorrichtung nach einem der Ansprüche 9 bis 13, **dadurch gekennzeichnet, dass** die Zugabeeinrichtungen (10) als Austrittsöffnungen oder Düsen ausgebildet sind und vorzugsweise mittels Dosierpumpen, beispielsweise Zahnradpumpen oder Membranpumpen, beschickbar sind und insbesondere so ausgebildet sind, dass die Zusatzstoffe in Tröpfchenform eindosierbar sind.

15. Vorrichtung nach einem der Ansprüche 9 bis 14, **dadurch gekennzeichnet, dass** die Zugabeeinrichtungen (10) bündig mit der Innenwand des Behälters (1) abschließen und insbesondere nicht von der Innenseite des Behälter (1) ins Innere des Behälters (1) vor- bzw. abstehen.

16. Vorrichtung nach einem der Ansprüche 9 bis 15, **dadurch gekennzeichnet, dass,** unabhängig von bzw. zusätzlich zu der Erwärmung des Materials durch beispielsweise die Mischwerkzeuge (4) oder weiteren das Material erwärmenden Erwärmungsvorrichtungen, zumindest eine separate Heizeinrichtung vorgesehen ist, mit der, insbesondere ausschließlich, die Innenseite bzw. die Seitenwand des Behälters (1), und gegebenenfalls auch die Zugabeeinrichtungen (10) und/oder deren Zuleitungen bzw. Vorratsbehälter, separat beheizbar sind, um die Viskosität der zugegebenen Zusatzstoffe zu vermindern und die Benetzung an der Seitenwand des Behälters (1) zu erhöhen.

17. Vorrichtung nach einem der Ansprüche 9 bis 16, **dadurch gekennzeichnet, dass** die Oberfläche der Innenseite des Behälters (1), insbesondere durch Aufbringung einer Anti-Haft-Beschichtung, einer Prägung, etc., als nicht benetzbare Oberfläche ausgebildet ist bzw. so ausgestaltet ist, dass die Benetzbarkeit minimiert bzw. keine Benetzung der Innenseite durch die zugegebenen Zusatzstoffe erfolgt.

18. Vorrichtung nach einem der Ansprüche 9 bis 17, **dadurch gekennzeichnet, dass** mehrere Aufnahmebehälter (1), insbesondere aufeinanderfolgend, vorgesehen sind und die Zugabeeinrichtungen (10) zumindest im ersten Aufnahmebehälter (1) angeordnet sind.

## Claims

1. A method for introducing and adding non-dry particulate, particularly of non-dry powdery, additives or coating stuffs of a liquid, solid, semi-solid or pasty consistency, optionally in a suspended or emulsified form, in particular of an elevated viscosity, such as peroxides, fats, waxes, IV enhancers, polymers or the like, to a material that is moved, mixed and optionally heated and comminuted, in a receiving receptacle or cutting condenser (1), and is present in a lumpy or particulate form, particularly polymeric particles or flakes, wood fibres, scraps of paper or the like, said additives being added below the level of the material or of the material particles present in said receptacle (1), **characterised in that** said additives, particularly very highly viscous additives, are added in that region or at that height of said receptacle (1), where the material particles, which in particular rotate inside said receptacle (1), exert the highest pressure to the lateral wall of said receptacle (1).

2. Method according to claim 1, **characterised in that** said additives are added in the middle third region of the charging level of the material within the receptacle (1) or of a mixing spout formed by rotation.

3. Method according to claim 1 or 2, **characterised in that** said additives are added through one or more adding means (10) which are arranged at the inner side of the lateral wall of said receptacle (1) or which discharge through said lateral wall, and which, in particular are either distributed over the circumference at the same level, or are superposed in a line, and are formed as adding openings or nozzles, and are optionally supplied by metering pumps, for example gear pumps or diaphragm pumps.

4. Method according to any of claims 1 to 3, **characterised in that** the level of the material particles or of the mixing spout formed by the movement within said receptacle (1) is maintained in such a way, that it is always above said adding means (10).

5. Method according to any of claims 1 to 4, **characterised in that** said additives are entrained or introduced by means of the material particles passing or rotating along the inner side of said lateral wall of said receptacle (1) and flowing over said adding means (10), or that said additives are deposited in this way onto said material particles.

6. Method according to any of claims 1 to 5, **characterised in that** prior to and/or during adding said additives, said inner side or the lateral wall of said receptacle (1) is additionally and separately heated, independent from the temperature of the material present within said receptacle (1), in order to reduce viscosity of the supplied additives and to enhance wetting.

7. Method according to any of claims 1 to 6, **characterised in that** said additives, particularly reactive additives, in dependence on the degree of dilution by an optional carrier of said additives, are added in an amount of between 0.01 and 20 % by weight with respect to the total weight of the final product.

8. Method according to any of claims 1 to 7, **characterised in that** said additives are added in maximum up to an amount necessary for wetting the total surface area of the material or the material particles present within said receptacle (1).

9. A device for carrying out the method according to any of claims 1 to 8, comprising at least one receiving receptacle or cutting condenser (1), in which at least one mixing tool (4) is arranged, which in particular is rotatable about a vertical axis, and which moves or rotates, mixes, heats and/or optionally comminutes the lumpy or particulate material to be treated, particularly plastic material in the form of non-molten polymeric particles, wood fibres, scraps of paper or the like, wherein at least one adding means (10) for non-dry powdery additives of a liquid, solid, semi-solid or pasty consistency, optionally in a suspended or emulsified form, in particular of an elevated viscosity, for example peroxides, fats, waxes, IV enhancers, polymers or the like is provided, wherein said adding means (10) is/are arranged below the level of the material or of the material particles present within said receptacle (1) during operation, **characterised in that** said adding means (10) are arranged **in that** region of said receptacle (1), where said material particles, being preferably moved and in particular being rotated within said receptacle (1), exert the highest pressure onto the lateral wall of said receptacle (1).

10. Device according to claim 9, **characterised in that** said adding means (10) are arranged at the inner side of said lateral wall of said receptacle (1) or discharge into said receptacle (1) through said lateral wall, or are formed in the lateral wall of said receptacle (1).

11. Device according to claim 9 or 10, **characterised in that** said adding means (10) are preferably evenly, distributed over the circumference of said lateral wall of said receptacle (1) at the same height or are superposed in a line one above the other.

12. Device according to any of claims 9 to 11, **characterised in that** said adding means (10) within said receptacle (1) are arranged at a height and at a distance from the bottom or from the mixing tool (4), where said adding means (10) are always below the charging level, predetermined by the method, of said material particles, which are present within said receptacle (1) or rotate in it, or below the level of a mixing spout formed by moving or rotating said material particles.

13. Device according to any of claims 9 to 12, **characterised in that** said adding means (10) are arranged at the height of the middle third region of the charging level, predetermined by the method, of the material within the receptacle (1) or of the mixing spout.

14. Device according to any of claims 9 to 13, **characterised in that** said adding means (10) are formed as outlet openings or nozzles, and may preferably be fed by means of metering pumps, for example gear pumps or diaphragm pumps, and are, in particular, formed so as to be able to meter said additives in the form of droplets.

15. Device according to any of claims 9 to 14, **characterised in that** said adding means (10) are flush with the inner wall of said receptacle (1) and, in particular, do not protrude or project from the inner side of said receptacle (1) towards the interior of said receptacle (1).

16. Device according to any of claims 9 to 15, **characterised in that**, independently from or in addition to heating the material, for example, by the mixing tools (4) or other heating means, that heat up said material, at least one separate heating device is provided, for example, by the mixing tools (4) or other heating means, that heat up said material, by which the inner side or the lateral wall of said receptacle (1), and optionally also the adding means (10) and/or their feeding conduits or the storing receptacle, may separately be heated so as to reduce the viscosity of the added additives, and to enhance wetting at the lateral wall of said receptacle (1).

17. Device according to any of claims 9 to 16, **characterised in that** the surface of the inner side of said receptacle (1) is formed as a non-wettable surface, particularly by applying anti-adhesion coating, by embossing or the like, or is equipped in a way that wettability is minimised or no wetting of the inner side occurs by the additives added.

18. Device according to any of claims 9 to 17, **characterised in that** several receiving receptacles (1) are provided, particularly one following the other, and that said adding means (10) are arranged at least within the first receiving receptacles (1).

## Revendications

1. Procédé d'introduction et d'addition des additifs ou des matériaux de revêtement en forme des particules non-sèches, particulièrement en forme de poudre, ayant une consistance liquide, solide, semi-solide ou pâteuse, le cas échéant en forme de suspension ou d'émulsion, en particulier ayant une viscosité élevée, comme par exemple des peroxides, des graisses, des cires, des agents d'amélioration de la VI, des polymères ou pareil, à un matériau agité, mêlé et, le cas échéant, réchauffé et concassé, présent en forme des morceaux ou des particules dans un réservoir de réception ou compresseur/coupeur (1), particulièrement des particules ou écailles de polymère, des fibres ligneuses, du papier déchiqueté ou pareil, dans lequel les additifs sont ajoutés au-dessous du niveau du matériau ou des particules du matériau présent(s) dans le réservoir (1), **caractérisé en ce, que** les additifs, en particulier les additifs très visqueux, sont ajoutés à cette zone ou à ce niveau du réservoir (1), où les particules de matériau, étant en particulier en rotation dans le réservoir (1), exercent la pression la plus grande à la paroi latérale du réservoir (1).

2. Procédé selon la revendication 1, **caractérisé en ce, que** les additifs son ajoutés à la zone de tiers médiane du niveau de remplissage du matériau dans le réservoir (1) ou à une trombe de mélange formée par la rotation.

3. Procédé selon la revendication 1 ou 2, les additifs sont ajoutés à travers un ou plusieurs dispositif(s) d'addition (10), qui est/sont disposé(s) au côté intérieur de la paroi latérale du réservoir (1) ou débouche(nt) à travers la paroi latérale, qui sont, en particulier, distribué(s) soit sur la circonférence à un niveau égal, soit superposés en série, et qui sont formé comme des ouvertures d'addition ou comme des buses, le cas échéant alimenté(s) par des pompes de dosage, par exemple des pompes à engrenage ou des pompes à diaphragme.

4. Procédé selon une quelconque des revendications 1 à 3, **caractérisé en ce, que** le niveau des particules de matériau ou de la trombe de mélange formée par le mouvement dans le réservoir (1) est maintenu de forme, qu'il est toujours au-dessus du/des dispositif(s) d'addition (10).

5. Procédé selon une quelconque des revendications 1 à 4, **caractérisé en ce, que** les additifs sont entraînés ou introduits par les et au moyens des particules de matériau, qui passent le long du côté intérieur de la paroi latérale du réservoir (1) et les dispositif(s) d'addition (10) ou que les additifs sont appliqués de cette manière aux particules de matériau.

6. Procédé selon une quelconque des revendications 1 à 5, **caractérisé en ce, qu'**avant de et/ou pendant l'addition des additifs on chauffe additionnellement et séparément le côté intérieur et la paroi latérale du réservoir (1) indépendamment de la température du matériau présent dans le réservoir (1), pour réduire la viscosité des additifs amenés et pour augmenter le mouillage.

7. Procédé selon une quelconque des revendications 1 à 6, **caractérisé en ce, que** les additifs, particulièrement les additifs réactifs, sont ajoutés en quantités entre 0,01 et 20 % en poids, par rapport au poids total du produit final, selon le degré de dilution par un support des additifs présent le cas échéant.

8. Procédé selon une quelconque des revendications 1 à 7, **caractérisé en ce, que** les additifs sont ajoutés en maximum jusqu'à une quantité nécessaire pour mouiller la surface totale du matériau ou des particules de matériau présent(s) dans le réservoir (1).

9. Dispositif pour l'exécution du procédé selon une quelconque des revendications 1 à 8, comprenant au moins un réservoir de réception ou compresseur/coupeur (1), dans lequel au moins un outil mélangeur (4) est disposé, en particulier tournant autour d'un axe vertical, qui meut ou met en rotation le matériau à traiter en forme des morceaux ou des particules, particulièrement un matériau synthétique en forme de particules de polymère pas en fusion, des fibres ligneuses, du papier déchiqueté ou pareil, que l'on le mélange, le chauffe ou, le cas échéant, le concasse, au moins un dispositif d'addition (10) étant prévu pour des additifs non-sèches en forme de particules, ayant une consistance liquide, solide, semi-solide ou pâteuse, le cas échéant en forme de suspension ou d'émulsion, en particulier ayant une viscosité élevée, par exemple des peroxides, des graisses, des cires, des agents d'amélioration de la VI, des polymères ou pareil, dans lequel le dispositif d'addition (10) est disposé au-dessous du niveau du matériau ou des particules de matériau présent dans le réservoir (1) pendant son fonctionnement, **caractérisé en ce, que** les dispositifs d'addition (10) sont disposés dans cette zone du réservoir (1), où les particules de matériau, préférablement en mouvement, en particulier étant en rotation dans le réservoir (1), exercent la pression la plus grande à la paroi latérale du réservoir (1).

10. Dispositif selon la revendication 9, **caractérisé en ce, que** les dispositifs d'addition (10) sont disposés au côté intérieur de la paroi latérale du réservoir (1) ou débouchent ou sont formés dans le réservoir (1) à travers la paroi latérale du réservoir (1).

11. Dispositif selon la revendication 9 ou 10, **caractérisé en ce, que** les dispositifs d'addition (10) sont distribués, préférablement d'une manière uniforme, sur la circonférence à un niveau égal ou sont superposés en série l'un au-dessus de l'autre.

12. Dispositif selon une quelconque des revendications 9 à 11, **caractérisé en ce, que** les dispositifs d'addition (10) sont disposés dans le réservoir à un niveau ou à une distance du fond ou de l'outil de mélangeur (4), auquel les dispositifs d'addition (10) sont toujours au-dessous du niveau de remplissage, donné par le procédé, des particule de matériau présent ou en rotation dans le réservoir (1) ou du niveau de la trombe de mélange formée lors du mouvement ou de la rotation des particules de matériau.

13. Dispositif selon une quelconque des revendications 9 à 12, **caractérisé en ce, que** les dispositifs d'addition (10) sont disposés au niveau de la zone de tiers médiane du niveau de remplissage, donné par le procédé, du matériau dans le réservoir (1) ou de la trombe de mélange.

14. Dispositif selon une quelconque des revendications 9 à 13, **caractérisé en ce, que** les dispositifs d'addition (10) sont formés comme des ouvertures de sortie ou comme des buses, préférablement capable à être alimentés par des pompes de dosage, par exemple des pompes à engrenage ou des pompes à diaphragme, et sont formés en particulier d'une manière, que les additifs peuvent être dosés en forme de gouttelettes.

15. Dispositif selon une quelconque des revendications 9 à 14, **caractérisé en ce, que** les dispositifs d'addition (10) sont à fleur de la paroi intérieure du réservoir (1) et, en particulier, ne font pas saillie du côté intérieur du réservoir (1) vers l'intérieur du réservoir (1).

16. Dispositif selon une quelconque des revendications 9 à 15, **caractérisé en ce, qu'**au moins un dispositif de chauffage séparé est prévu, indépendamment du ou additionnellement au chauffage du matériau, par exemple par les outils de mélange (4) ou des autres dispositifs de chauffage, qui chauffent le matériau, par lequel le côté intérieur ou la paroi latérale du réservoir (1), le cas échéant les dispositifs d'addition (10) et/ou les conduits d'alimentation de ceux-ci et/ou le réservoir de stockage, peut/peuvent être chauffée(s) d'une manière séparée, particulièrement d'une façon exclusive, pour réduire la viscosité des additifs ajoutés et pour augmenter le mouillage à la paroi latérale du réservoir (1).

17. Dispositif selon une quelconque des revendications 9 à 16, **caractérisé en ce, que** la surface du côté intérieur du réservoir (1) est formée comme une surface non mouillable, particulièrement par application d'une couche antiadhésive, par une frappe etc., ou qu'elle est dotée d'une façon, que la mouillabilité est minimalisée ou qu'un mouillage du côté intérieur ne s'effectue pas par les additifs ajoutés.

18. Dispositif selon une quelconque des revendications 9 à 17, **caractérisé en ce, que** plusieurs réservoirs de réception (1) sont prévus, particulièrement en séries, et que les dispositifs d'addition (10) sont au moins disposés au premier réservoir de réception (1).
